# EUROPEAN PATENT APPLICATION

(11) **EP 4 474 500 A1**
(43) Date of publication of application: **11.12.2024**
(21) Application number: 23749470.3
(22) Date of filing: 05.01.2023
(51) Int. Cl.: C22B 3/44, C02F 1/62, C07C 211/18, C07D 307/14, C22B 11/00

(54) **PLATINUM-GROUP METAL RECOVERY AGENT AND PLATINUM-GROUP METAL RECOVERY METHOD**

(30) Priority: 01.02.2022 JP 2022014362
(71) Applicant: MITSUBISHI GAS CHEMICAL COMPANY, INC., Chiyoda-ku Tokyo 100-8324 (JP)
(72) Inventor: MATSUMOTO, Kazuya, Akita-shi, Akita 010-8502 (JP); JIKEI, Mitsutoshi, Akita-shi, Akita 010-8502 (JP)
(74) Representative: Hoffmann Eitle
(86) International application number: PCT/JP2023/000098
(87) International publication number: WO 2023/149153

(57) **Abstract**

Provided are a platinum-group metal recovery agent represented by the following general Formula (1) and a platinum-group metal recovery method for recovering platinum from a hydrochloric acid solution containing platinum using the platinum-group metal recovery agent. (In Formula (1), X is a methylene group, an oxygen atom, or an ethylene group; n is 3 when X is a methylene group; and n is 2 when X is an oxygen atom or an ethylene group.)

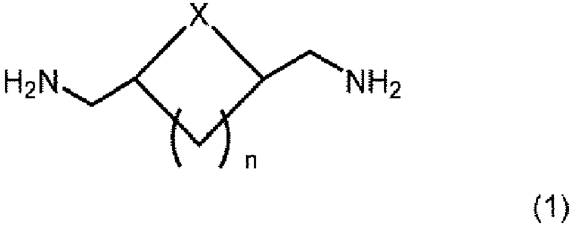

## Description

### Technical Field

The present invention relates to a platinum-group metal recovery agent and a platinum-group metal recovery method.

### Background Art

Platinum-group metals are of great industrial importance. Among them, platinum, palladium, and rhodium are mainly used as automobile exhaust gas purifying catalysts. Therefore, it is important to selectively separate and recover platinum-group metals from spent catalysts.

In general, methods such as an electrolytic deposition method, a cementation method, an ion exchange method, a precipitation method, and a solvent extraction method are used for recovery of noble metals. Among these methods, the precipitation method and the solvent extraction method, which are excellent in economical efficiency and operability, are widely used.

As a method for selectively recovering a platinum-group metal by the precipitation method, a method using an amine has been studied.

For example, Patent Document 1 discloses a rhodium recovery agent that has an amide bond and a phenylenediamine structure and is a solid insoluble in hydrochloric acid at a specific concentration for the purpose of efficiently recovering rhodium at low cost.

Patent Document 2 discloses that a platinum-group metal can be precipitated and recovered by adding a compound having a diaminodiphenyl structure to a hydrochloric acid solution containing the platinum-group metal, particularly for the purpose of efficiently separating rhodium.

### Citation List

### Patent Document

Patent Document 1: JP 2017-179409 A
Patent Document 2: JP 2019-206747 A

### Summary of Invention

### Technical Problem

In the recovery of platinum-group metals by the known precipitation method or solvent extraction method, palladium is preferentially recovered, and it is difficult to selectively recover platinum and rhodium first. Although Patent Documents 1 and 2 describe methods of recovering rhodium, platinum cannot be preferentially and selectively recovered by the methods. Therefore, there is a demand for a method capable of preferentially and selectively recovering platinum even in the presence of palladium and rhodium.

Accordingly, an object of the present invention is to provide a platinum-group metal recovery agent and a platinum-group metal recovery method, which are capable of selectively recovering platinum at a high recovery rate.

### Solution to Problem

The present inventors have found that a specific aliphatic diamine is excellent as a platinum-group metal recovery agent. Further, the present inventors have found a method capable of selectively recovering platinum at a high recovery rate by using the platinum-group metal recovery agent. Furthermore, the present inventors have found a method capable of preferentially and selectively recovering platinum even in the presence of palladium and rhodium, which has been difficult in the past, and have completed the present invention.

Accordingly, the present invention provides a platinum-group metal recovery agent represented by the following general Formula (1) and a platinum-group metal recovery method for recovering platinum from a hydrochloric acid solution containing platinum using the platinum-group metal recovery agent.

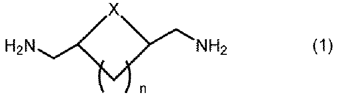

(In Formula (1), X is a methylene group, an oxygen atom, or an ethylene group; n is 3 when X is a methylene group; and n is 2 when X is an oxygen atom or an ethylene group.)

### Advantageous Effects of Invention

The present invention can provide a platinum-group metal recovery agent and a platinum-group metal recovery method, which are capable of selectively recovering platinum at a high recovery rate.

### Description of Embodiments

### Platinum-group metal recovery agent

The platinum-group metal recovery agent of the present invention is represented by the following general Formula (1):

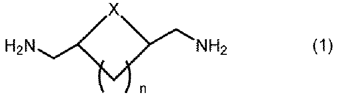

(In Formula (1), X is a methylene group, an oxygen atom, or an ethylene group; n is 3 when X is a methylene group; and n is 2 when X is an oxygen atom or an ethylene group.)

The compound represented by general Formula (1) above can be used as a platinum-group metal recovery agent.

Specifically, the platinum-group metal recovery agent of the present invention is at least one selected from the group consisting of 1,4-bis(aminomethyl)cyclohexane (1,4-BAC), 1,3-bis(aminomethyl)cyclohexane (1,3-BAC), and 2,5-bis(aminomethyl)tetrahydrofuran (H-AMF).

Specifically, they are compounds represented by the following formulae.

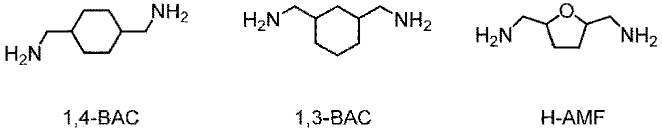

Among the compounds represented by Formula (1) above, the platinum-group metal recovery agent of the present invention is preferably at least one selected from the group consisting of 1,4-bis(aminomethyl)cyclohexane and 2,5-bis(aminomethyl)tetrahydrofuran, and more preferably 1,4-bis(aminomethyl)cyclohexane.

Platinum can be selectively recovered at a high recovery rate by using the compound represented by Formula (1), especially 1,4-bis(aminomethyl)cyclohexane or 2,5-bis(aminomethyl)tetrahydrofuran as the platinum-group metal recovery agent. Furthermore, platinum can be preferentially and selectively recovered even in the presence of palladium and rhodium, which has been difficult in the past.

The reason why platinum can be selectively recovered by using the platinum-group metal recovery agent of the present invention is not clear, but is considered to be because, when the specific aliphatic diamine, which is the platinum-group metal recovery agent of the present invention, is mixed with a hydrochloric acid solution of platinum-group metals, the specific aliphatic diamine is likely to form crystals with platinum to thereby allow for selective precipitation of platinum.

For the platinum-group metal recovery agent represented by Formula (1) above, one type may be used alone, or two or more types may be used in combination.

Furthermore, 1,4-bis(aminomethyl)cyclohexane contains a trans form (trans isomer) and a cis form (cis isomer), and preferably contains a trans form in a higher proportion. That is, the platinum-group metal recovery agent of the present invention is more preferably 1,4-bis(aminomethyl)cyclohexane containing a trans form in a proportion of more than 50%, even more preferably 1,4-bis(aminomethyl)cyclohexane containing a trans form in a proportion of 60% or more, and yet even more preferably 1,4-bis(aminomethyl)cyclohexane containing a trans form in a proportion of 70% or more. The upper limit of the proportion of the trans form is not limited, and may be any value as long as it is 100% or lower. When the 1,4-bis(aminomethyl)cyclohexane containing a trans form in a high proportion is used, platinum can be efficiently and selectively precipitated and recovered by using a smaller amount of the platinum-group metal recovery agent.

In the present specification, the 1,4-bis(aminomethyl)cyclohexane containing a trans form in a high proportion (generally, the proportion of the trans form is 60% or more) may be referred to as 1,4-BACT.

The platinum-group metal recovery agent of the present invention is a compound represented by Formula (1) above and is a specific aliphatic diamine, but may also be in a form of hydrohalic acid salt of the diamine. The hydrohalic acid salt is preferably a hydrochloride. When the platinum-group metal recovery agent of the present invention is a hydrochloride, heat generation or a change in hydrochloric acid concentration due to a neutralization reaction can be prevented when the platinum-group metal recovery agent is mixed with a hydrochloric acid solution containing a platinum-group metal.

The hydrochloric acid concentration (HCl concentration) of the hydrochloric acid solution used in the platinum-group metal recovery method of the present specification is a hydrochloric acid concentration after mixing of the platinum-group metal recovery agent of the present invention. However, when a hydrochloride of the compound represented by Formula (1) above is used as the platinum-group metal recovery agent of the present invention, the hydrochloric acid concentration does not change, and thus the hydrochloric acid concentration may be a hydrochloric acid concentration before mixing of the platinum-group metal recovery agent of the present invention.

### Platinum-group metal recovery method (recovery of platinum)

The platinum-group metal recovery method of the present invention is intended to selectively recover platinum, and can preferentially recover platinum.

The platinum-group metal recovery method of the present invention is a method of recovering platinum from a hydrochloric acid solution containing platinum using the platinum-group metal recovery agent described above. The platinum-group metal recovery method of the present invention is preferably a method involving mixing the platinum-group metal recovery agent with the hydrochloric acid solution, thereby precipitating platinum.

According to the platinum-group metal recovery method of the present invention, platinum can be recovered from a hydrochloric acid solution containing only platinum as a platinum-group metal, and platinum can be selectively recovered even when the hydrochloric acid solution contains at least one selected from the group consisting of palladium and rhodium. In addition, platinum can be selectively recovered even when the hydrochloric acid solution contains palladium. Furthermore, platinum can be selectively recovered even when the hydrochloric acid solution contains both palladium and rhodium.

A specific method will be described below.

Firstly, the platinum-group metal recovery agent is added to a hydrochloric acid solution containing at least platinum.

By using the platinum-group metal recovery agent of the present invention, platinum can be selectively recovered from hydrochloric acid solutions having various hydrochloric acid concentrations. The hydrochloric acid concentration of the hydrochloric acid solution containing platinum is preferably from 1 to 12 mol/L, more preferably from 4 to 10 mol/L, and even more preferably from 5 to 9 mol/L.

When the platinum-group metal recovery agent is added, a molar ratio of the platinum-group metal recovery agent to platinum contained in the hydrochloric acid solution; i.e., [platinum-group metal recovery agent/platinum (mol/mol)] is preferably 1 or more, more preferably 2 or more, and even more preferably 5 or more. Furthermore, a molar ratio of the platinum-group metal recovery agent to platinum contained in the hydrochloric acid solution; i.e., [platinum-group metal recovery agent/platinum (mol/mol)] is preferably 200 or less, more preferably 150 or less, even more preferably 100 or less, and yet even more preferably 20 or less.

The phrase "a molar ratio of the platinum-group metal recovery agent to platinum contained in the hydrochloric acid solution; i.e., [platinum-group metal recovery agent/platinum (mol/mol)] is 1 or more" means "adding the platinum-group metal recovery agent in such an amount that the molar ratio of the platinum-group metal recovery agent to platinum contained in the hydrochloric acid solution [platinum-group metal recovery agent/platinum (mol/mol)] is 1 or more".

Especially in a case where the 1,4-bis(aminomethyl)cyclohexane (1,4-BAC) is used as the platinum-group metal recovery agent, platinum can be selectively recovered at a high recovery rate even with a small addition amount of the platinum-group metal recovery agent with respect to the amount of platinum, and thus the molar ratio of the platinum-group metal recovery agent to platinum contained in the hydrochloric acid solution; i.e., [platinum-group metal recovery agent/platinum (mol/mol)] is preferably from 1 to 100, more preferably from 2 to 50, even more preferably from 5 to 20, and yet even more preferably from 5 to 10.

Furthermore, in a case where the 1,4-bis(aminomethyl)cyclohexane having a trans form proportion of 60% or more (1,4-BACT) is used, platinum can be selectively recovered at a high recovery rate even with a smaller addition amount of the platinum-group metal recovery agent with respect to the amount of platinum, and thus the molar ratio of the platinum-group metal recovery agent to platinum contained in the hydrochloric acid solution; i.e., [platinum-group metal recovery agent/platinum (mol/mol)] is preferably from 1 to 100, more preferably from 1 to 30, even more preferably from 1 to 10, and yet even more preferably from 2 to 8.

When the molar ratio is within the above range, the recovery rate of platinum increases, rhodium and palladium are not likely to be contained in the precipitate, and platinum can be recovered with high purity.

The method for mixing the platinum-group metal recovery agent and the hydrochloric acid solution is not limited, and platinum is precipitated by adding the platinum-group metal recovery agent into the hydrochloric acid solution and stirring or shaking the mixture for a predetermined time.

When the platinum-group metal recovery agent of the present invention is used, precipitation rapidly occurs after mixing of the platinum-group metal recovery agent and the hydrochloric acid solution, and thus platinum can be recovered within a short period of time. The time for stirring or shaking is preferably 1 minute or longer, and more preferably 5 minutes or longer. The upper limit is not limited, but the time for stirring or shaking is preferably 10 hours or shorter, and more preferably 5 hours or shorter, from the viewpoint of efficiency. The time for stirring or shaking may be appropriately adjusted depending on the concentration of each component or temperature.

The temperature at the time of stirring or shaking may be from 0 to 100°C. According to the present invention, platinum can be selectively recovered from a hydrochloric acid solution at any temperature. From the viewpoint of recovery rate, the temperature at the time of stirring or shaking is preferably from 10 to 70°C, more preferably from 10 to 50°C, and even more preferably from 10 to 35°C.

The precipitate containing platinum obtained in the above manner can be recovered by a solid-liquid separation process such as filtration or centrifugation.

Since the precipitate contains platinum and also contains the platinum-group metal recovery agent which is an aliphatic diamine, etc., platinum can be obtained in the form of a metal or an oxide by firing the precipitate in air. The firing temperature is preferably from 400 to 1000°C, and more preferably from 500 to 700°C. The firing time is preferably from 5 to 100 minutes, and more preferably from 10 to 80 minutes. By adjusting these firing conditions, platinum can be obtained in the form of a metal, preferably a simple metal.

### Platinum-group metal purification method (purification of platinum)

The platinum-group metal recovery agent described above can also be used for purification of platinum.

The platinum-group metal purification method of the present invention is a method of purifying platinum by removing a metal as an impurity from a hydrochloric acid solution containing platinum as a main component by using the platinum-group metal recovery agent described above.

The "hydrochloric acid solution containing platinum as a main component" used in the platinum-group metal purification method of the present invention refers to a hydrochloric acid solution which contains platinum and a metal (impurity) other than platinum and in which the molar content of platinum is the largest among the metals contained in the hydrochloric acid solution.

The hydrochloric acid solution containing platinum as a main component may be obtained by dissolving a solid containing platinum in hydrochloric acid or may be obtained by adding hydrochloric acid in an aqueous solution containing platinum. Preferably, the hydrochloric acid solution containing platinum as a main component is obtained by dissolving, in hydrochloric acid, a precipitate obtained by the platinum-group metal recovery method described above in the section "Platinum-group metal recovery method (recovery of platinum)".

In the platinum-group metal purification method of the present invention, purification is preferably performed under the same conditions as in the platinum-group metal recovery method described above in the section "Platinum-group metal recovery method (recovery of platinum)". That is, the platinum-group metal purification method of the present invention is preferably a method involving mixing the platinum-group metal recovery agent with the hydrochloric acid solution, thereby precipitating platinum. Details will be described below.

Firstly, the platinum-group metal recovery agent is added to a hydrochloric acid solution containing platinum as a main component.

By using the platinum-group metal recovery agent of the present invention, platinum can be purified even when hydrochloric acid solutions having various hydrochloric acid concentrations are used. The hydrochloric acid concentration of the hydrochloric acid solution containing platinum as a main component is preferably from 1 to 12 mol/L, more preferably from 4 to 10 mol/L, and even more preferably from 5 to 9 mol/L.

When the platinum-group metal recovery agent is added, the molar ratio of the platinum-group metal recovery agent to platinum contained in the hydrochloric acid solution; i.e., [platinum-group metal recovery agent/platinum (mol/mol)] is preferably 1 or more, more preferably 2 or more, and even more preferably 5 or more. Furthermore, the molar ratio of the platinum-group metal recovery agent to platinum contained in the hydrochloric acid solution; i.e., [platinum-group metal recovery agent/platinum (mol/mol)] is preferably 200 or less, more preferably 150 or less, even more preferably 100 or less, and yet even more preferably 20 or less.

Especially in a case where the 1,4-bis(aminomethyl)cyclohexane (1,4-BAC) is used as the platinum-group metal recovery agent, platinum can be efficiently purified even with a small addition amount of the platinum-group metal recovery agent with respect to the amount of platinum, and thus the molar ratio of the platinum-group metal recovery agent to platinum contained in the hydrochloric acid solution; i.e., [platinum-group metal recovery agent/platinum (mol/mol)] is preferably from 1 to 100, more preferably from 2 to 50, even more preferably from 5 to 20, and yet even more preferably from 5 to 10.

Furthermore, in a case where the 1,4-bis(aminomethyl)cyclohexane having a trans form proportion of 60% or more (1,4-BACT) is used, platinum can be efficiently purified even with a smaller addition amount of the platinum-group metal recovery agent with respect to the amount of platinum, and thus the molar ratio of the platinum-group metal recovery agent to platinum contained in the hydrochloric acid solution; i.e., [platinum-group metal recovery agent/platinum (mol/mol)] is preferably from 1 to 100, more preferably from 1 to 30, even more preferably from 1 to 10, and yet even more preferably from 2 to 8.

When the molar ratio is in the range described above, even when rhodium or palladium is contained as an impurity metal, rhodium or palladium is less likely to be contained in the resulting precipitate, and thus platinum with a high purity can be obtained.

The method for mixing the platinum-group metal recovery agent and the hydrochloric acid solution is not limited, and platinum is precipitated by adding the platinum-group metal recovery agent into the hydrochloric acid solution and stirring or shaking the mixture for a predetermined time.

When the platinum-group metal recovery agent is used, precipitation rapidly occurs after mixing of the platinum-group metal recovery agent and the hydrochloric acid solution, and thus platinum can be purified within a short period of time. The time for stirring or shaking is preferably 1 minute or longer, and more preferably 5 minutes or longer. The upper limit is not limited, but the time for stirring or shaking is preferably 10 hours or shorter, and more preferably 5 hours or shorter, from the viewpoint of efficiency. The time for stirring or shaking may be appropriately adjusted depending on the concentration of each component or temperature.

The temperature at the time of stirring or shaking may be from 0 to 100°C, and platinum can be purified by using a hydrochloric acid solution at any temperature. However, from the viewpoint of recovery rate, the temperature at the time of stirring or shaking is preferably from 10 to 70°C, more preferably from 10 to 50°C, and even more preferably from 10 to 35°C.

The precipitate containing platinum obtained in the above manner can be recovered by a solid-liquid separation process such as filtration or centrifugation.

Since the precipitate contains platinum and also contains the platinum-group metal recovery agent which is an aliphatic diamine, etc., platinum can be obtained in the form of a metal or an oxide by firing the precipitate in air. The firing temperature is preferably from 400 to 1000°C, and more preferably from 500 to 700°C. The firing time is preferably from 5 to 100 minutes, and more preferably from 10 to 80 minutes. By adjusting these firing conditions, platinum can be obtained in the form of a metal, preferably a simple metal.

### Examples

The present invention will be described in detail based on Examples shown below, but the present invention is not limited to these Examples.

### Recovery of platinum with 1,4-bis(aminomethyl)cyclohexane

### Example 1: Influence of hydrochloric acid concentration

To each hydrochloric acid solution containing 5 mmol/L of palladium, 5 mmol/L of platinum, and 5 mmol/L of rhodium and having a hydrochloric acid concentration shown in Table 1, 1,4-bis(aminomethyl)cyclohexane (1,4-BAC; trans form: 33%; cis form: 67%) was added as a platinum-group metal recovery agent in an amount 15 times (mol/mol) the amount of platinum. The 1,4-bis(aminomethyl)cyclohexane used herein is in the form of hydrochloride. Thereafter, the mixture was shaken at 25°C for 30 minutes to yield a precipitate. The resultant precipitate and solution were subjected to solid-liquid separation by centrifugation. The solution obtained after the solid-liquid separation was analyzed by ICP, and the amount of the platinum-group metal contained in the precipitate was calculated.

Table 1 shows the relationship between the hydrochloric acid concentration and the metal recovery rate. As is clear from Table 1, platinum can be selectively recovered as a precipitate in a wide range of hydrochloric acid concentrations of 1 mol/L or more by using 1,4-bis(aminomethyl)cyclohexane (1,4-BAC) as the platinum-group metal recovery agent. The metal recovery rate is the percentage of a value obtained by dividing the amount of a metal contained in the recovered precipitate by the amount of the metal before addition of the platinum-group metal recovery agent. In the following Examples, the metal recovery rate was determined in the same manner as described above.

### [Table 1]

**Table 1**

| Hydrochloric acid concentration (mol/L) | Metal recovery rate (%) | | |
|---|---|---|---|
| | Pt (platinum) | Pd (palladium) | Rh (rhodium) |
| 1 | 82.6 | 0.0 | 0.2 |
| 2 | 84.2 | 0.6 | 0.5 |
| 3 | 85.6 | 0.2 | 0.7 |
| 4 | 87.3 | 0.2 | 0.4 |
| 5 | 91.2 | 0.5 | 1.1 |
| 6 | 92.7 | 0.2 | 0.8 |
| 7 | 92.9 | 0.5 | 1.0 |
| 8 | 93.6 | 0.4 | 1.0 |

### Example 2: Influence of temperature

A precipitate and a solution were obtained in the same manner as in Example 1 except that the hydrochloric acid concentration of the hydrochloric acid solution was adjusted to 8 mol/L, and the temperature of the hydrochloric acid solution was changed as shown in Table 2. The amount of the platinum-group metal contained in the precipitate was calculated.

Table 2 shows the relationship between the temperature and the metal recovery rate. As is clear from Table 2, platinum can be selectively recovered as a precipitate in a wide temperature range by using 1,4-bis(aminomethyl)cyclohexane (1,4-BAC) as the platinum-group metal recovery agent.

### [Table 2]

**Table 2**

| Temperature (°C) | Metal recovery rate (%) | | |
|---|---|---|---|
| | Pt (platinum) | Pd (palladium) | Rh (rhodium) |
| 25 | 93.6 | 0.4 | 1.0 |
| 40 | 90.8 | 1.6 | 2.2 |
| 60 | 87.0 | 0.6 | 2.5 |
| 80 | 84.6 | 0.7 | 2.3 |

### Example 3: Influence of molar ratio of platinum-group metal recovery agent to platinum

A precipitate and a solution were obtained in the same manner as in Example 1 except that the hydrochloric acid concentration of the hydrochloric acid solution was adjusted to 8 mol/L, and the amount of the platinum-group metal recovery agent (1,4-bis(aminomethyl)cyclohexane (1,4-BAC)) added was changed as shown in Table 3. The amount of the platinum-group metal contained in the precipitate was calculated. The amount of 1,4-BAC added is indicated in times by mole with respect to platinum.

Table 3 shows the relationship between the amount of 1,4-BAC added and the metal recovery rate. As is clear from Table 3, platinum can be selectively recovered regardless of the amount of 1,4-BAC added. As is also clear from Table 3, under the condition that the amount of 1,4-BAC added is 5 times (mol/mol) or more the amount of platinum, 85% or more of platinum can be recovered as a precipitate, the platinum recovery rate is very high, and platinum can be selectively recovered.

### [Table 3]

**Table 3**

| 1,4-BAC/Pt (mol/mol) | Metal recovery rate (%) | | |
|---|---|---|---|
| | Pt (platinum) | Pd (palladium) | Rh (rhodium) |
| 1 | 33.0 | 2.8 | 0.9 |
| 2 | 54.6 | 0.6 | 0.5 |
| 5 | 85.4 | 1.1 | 1.8 |
| 10 | 93.2 | 2.2 | 1.7 |
| 15 | 93.6 | 0.4 | 1.0 |
| 30 | 94.3 | 3.4 | 4.5 |
| 50 | 94.5 | 4.0 | 7.4 |
| 100 | 95.5 | 5.2 | 5.7 |

### Example 4: Influence of trans form proportion

A precipitate and a solution were obtained in the same manner as in Example 3 except that the 1,4-bis(aminomethyl)cyclohexane (1,4-BAC; trans form: 33%; cis form: 67%) was changed to 1,4-bis(aminomethyl)cyclohexane having a high trans form proportion (1,4-BACT; trans form: 93%; cis form: 7%). The amount of the platinum-group metal contained in the precipitate was calculated.

Table 4 shows the relationship between the amount of 1,4-BACT added and the metal recovery rate. As is clear from Table 4, when the 1,4-BACT having a high trans form proportion is used as the platinum-group metal recovery agent, even under the condition that the amount of the 1,4-BACT added is 2 times (mol/mol) the amount of platinum, 85% or more of platinum can be recovered as a precipitate, and it can be seen that, even with a small amount of addition, the platinum recovery rate is very high and platinum can be selectively recovered.

### [Table 4]

**Table 4**

| 1,4-BACT/Pt (mol/mol) | Metal recovery rate (%) | | |
|---|---|---|---|
| | Pt (platinum) | Pd (palladium) | Rh (rhodium) |
| 1 | 62.0 | 0.5 | 1.2 |
| 2 | 85.0 | 0.4 | 3.1 |
| 5 | 94.5 | 0.6 | 3.3 |
| 10 | 97.0 | 0.4 | 3.5 |
| 15 | 98.1 | 0.2 | 4.0 |
| 30 | 98.3 | 1.2 | 7.2 |
| 50 | 98.5 | 1.9 | 8.5 |
| 100 | 98.5 | 1.8 | 8.5 |

### Example 5: Influence of shaking time

A precipitate and a solution were obtained in the same manner as in Example 4 except that 1,4-bis(aminomethyl)cyclohexane (1,4-BACT; trans form: 93%; cis form: 7%) was added as the platinum-group metal recovery agent in an amount 15 times (mol/mol) the amount of platinum, and the shaking time was changed as shown in Table 5. The amount of the platinum-group metal contained in the precipitate was calculated.

Table 5 shows the relationship between the shaking time and the metal recovery rate. As is clear from Table 5, 95% or more of platinum can be recovered as a precipitate by the shaking for 1 minute or longer, the platinum recovery rate is very high, and platinum can be selectively recovered.

### [Table 5]

**Table 5**

| Shaking time (min) | Metal recovery rate (%) | | |
|---|---|---|---|
| | Pt (platinum) | Pd (palladium) | Rh (rhodium) |
| 1 | 96.6 | 0.1 | 3.6 |
| 5 | 98.2 | 0.2 | 3.8 |
| 10 | 98.2 | 0.1 | 4.1 |
| 20 | 98.3 | 0.1 | 2.6 |
| 30 | 98.1 | 0.2 | 4.0 |
| 60 | 98.4 | 0.1 | 3.2 |

### Recovery of platinum from catalyst leaching solution and purification of platinum

### Example 6: Recovery of platinum from catalyst leaching solution

On the assumption that platinum-group metals were recovered from used automobile exhaust gas purifying catalysts, platinum was recovered from a catalyst leaching solution containing various metals as shown in Table 6.

To the aforementioned catalyst leaching solution (containing hydrochloric acid, and the hydrochloric acid concentration was 8 mol/L), 1,4-bis(aminomethyl)cyclohexane (1,4-BACT; trans form: 93%; cis form: 7%) was added as a platinum-group metal recovery agent in an amount 15 times (mol/mol) the amount of platinum. The 1,4-bis(aminomethyl)cyclohexane used herein is in the form of hydrochloride. Thereafter, the mixture was shaken at 25°C for 10 minutes to yield a precipitate. After the resultant precipitate was recovered by filtration, the precipitate was dissolved in 1 mol/L hydrochloric acid and analyzed by ICP, and the amounts of the metals contained in the precipitate were calculated.

The recovery rate of each metal after this recovery step is shown in Table 6. As is clear from Table 6, platinum can be selectively recovered as a precipitate from the catalyst leaching solution by using 1,4-bis(aminomethyl)cyclohexane as the platinum-group metal recovery agent. The purity of platinum contained in the precipitate was 87.0% when the total metal weight was taken as 100%.

In Table 6, "<0.1" means "less than 0.1".

### Example 7: Purification of platinum

Subsequently, platinum recovered from the catalyst leaching solution by the method described above was purified.

The precipitate recovered by the method described above was dissolved in 5 mol/L hydrochloric acid (platinum concentration: 500 mg/L). To the resultant hydrochloric acid solution, 1,4-bis(aminomethyl)cyclohexane (1,4-BACT; trans form: 93%; cis form: 7%) was added as a platinum-group metal recovery agent in an amount 15 times (mol/mol) the amount of platinum. The 1,4-bis(aminomethyl)cyclohexane used herein is in the form of hydrochloride. Thereafter, the mixture was shaken at 25°C for 10 minutes to yield a precipitate. After the resultant precipitate was recovered by filtration, the precipitate was dissolved in 1 mol/L hydrochloric acid and analyzed by ICP, and the amounts of the metals contained in the precipitate were calculated.

The recovery rate of each metal after this purification step is shown in Table 6. As is clear from Table 6, the amounts of palladium and rhodium contained in the precipitate significantly decrease after the purification step. When the total metal weight was taken as 100%, the purity of platinum contained in the precipitate was 99.3% after the purification step while the purity was 87.0% after the recovery step. As is clear from the results, when 1,4-bis(aminomethyl)cyclohexane is used as the platinum-group metal recovery agent, metals as impurities can be removed from the hydrochloric acid solution containing platinum as a main component, to thereby purify platinum.

### [Table 6]

**Table 6**

| Metal species | Initial metal concentration (mg/L) | Metal recovery rate after recovery step (%) | Metal recovery rate after purification step (%) |
|---|---|---|---|
| Pt (platinum) | 1516.0 | 99.2 | 95.6 |
| Pd (palladium) | 904.0 | 14.6 | 0.5 |
| Rh (rhodium) | 270.0 | 12.3 | <0.1 |
| Ce (cerium) | 62447.0 | <0.1 | <0.1 |
| Al (aluminum) | 1864.0 | 0.3 | 0.2 |
| Fe (iron) | 1544.0 | 2.6 | <0.1 |
| Zr (zirconium) | 328.0 | <0.1 | <0.1 |
| La (lanthanum) | 1237.0 | <0.1 | <0.1 |
| Mg (magnesium) | 23118.0 | <0.1 | <0.1 |
| Purity of platinum in precipitate (%) | - | 87.0 | 99.3 |

### Recovery of platinum-group metal with 2,5-bis(aminomethyl)tetrahydrofuran

### Example 8: Recovery of platinum-group metal

To 8 mol/L hydrochloric acid solution containing 5 mmol/L of palladium and 5 mmol/L of platinum, 2,5-bis(aminomethyl)tetrahydrofuran (H-AMF) was added as a platinum-group metal recovery agent in an amount 100 times (mol/mol) the amount of platinum. The 2,5-bis(aminomethyl)tetrahydrofuran used herein is in the form of hydrochloride. Thereafter, the mixture was shaken at 25°C for 30 minutes to yield a precipitate. The resultant precipitate and solution were subjected to solid-liquid separation by centrifugation. The solution obtained after the solid-liquid separation was analyzed by ICP, and the amount of the platinum-group metal contained in the precipitate was calculated.

As a result, 56% of platinum was recovered as a precipitate. In contrast, 7.2% of palladium was recovered as a precipitate. The results indicate that platinum can be selectively recovered as the precipitate.

## Claims

1. A platinum-group metal recovery agent represented by the following general Formula (1): 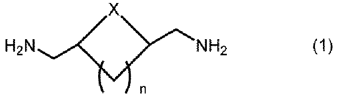 (in Formula (1), X is a methylene group, an oxygen atom, or an ethylene group; n is 3 when X is a methylene group; and n is 2 when X is an oxygen atom or an ethylene group).

2. The platinum-group metal recovery agent according to claim 1, which is at least one selected from the group consisting of 1,4-bis(aminomethyl)cyclohexane and 2,5-bis(aminomethyl)tetrahydrofuran.

3. The platinum-group metal recovery agent according to claim 1 or 2, which is 1,4-bis(aminomethyl)cyclohexane.

4. The platinum-group metal recovery agent according to claim 3, which contains a trans form in a proportion of 60% or more.

5. A platinum-group metal recovery method comprising recovering platinum from a hydrochloric acid solution containing platinum by using the platinum-group metal recovery agent described in any one of claims 1 to 4.

6. The platinum-group metal recovery method according to claim 5, which comprises mixing the platinum-group metal recovery agent with the hydrochloric acid solution to precipitate platinum.

7. The platinum-group metal recovery method according to claim 5 or 6, wherein the hydrochloric acid solution containing platinum contains at least one selected from the group consisting of palladium and rhodium.

8. The platinum-group metal recovery method according to any one of claims 5 to 7, wherein the hydrochloric acid solution containing platinum contains palladium.

9. The platinum-group metal recovery method according to any one of claims 5 to 8, wherein a molar ratio of the platinum-group metal recovery agent to platinum contained in the hydrochloric acid solution containing platinum [platinum-group metal recovery agent/platinum] is 2 or more.

10. A platinum-group metal purification method comprising removing a metal as an impurity from a hydrochloric acid solution containing platinum as a main component to purify platinum by using the platinum-group metal recovery agent described in any one of claims 1 to 4.

11. Use of a compound represented by the following general Formula (1) as a platinum-group metal recovery agent: 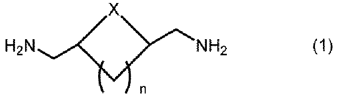 (in Formula (1), X is a methylene group, an oxygen atom, or an ethylene group; n is 3 when X is a methylene group; and n is 2 when X is an oxygen atom or an ethylene group).
